Europäisches Patentamt

(19) **European Patent Office**

Office européen des brevets

(11) Publication number: **0 228 185**
**B1**

(12) **EUROPEAN PATENT SPECIFICATION**

(45) Date of publication of the patent specification: **25.07.90**

(51) Int. Cl.⁵: **A61F 9/00**

(21) Application number: **86309208.6**

(22) Date of filing: **26.11.86**

(54) Tissue-implantable fluid-dissipating device.

(30) Priority: **27.11.85 US 802517**
**27.11.85 US 802574**

(43) Date of publication of application:
**08.07.87 Bulletin 87/28**

(45) Publication of the grant of the patent:
**25.07.90 Bulletin 90/30**

(84) Designated Contracting States:
**AT BE CH DE FR GB IT LI NL SE**

(56) References cited:
**FR-A- 2 569 987**
**US-A- 3 788 327**
**US-A- 4 240 434**
**US-A- 4 554 918**

**SOVIET INVENTIONS ILLUSTRATED, Section Mechanical, Week K42, 30th November 1983, abstract no. 83-793260 P32, Derwent Publications Ltd., London, GB; & SU - A - 980 711 (TURK EYE DISEASES) 15-12-1982**

(73) Proprietor: **White, Thomas C., 1127 Holly Drive, Sioux Falls South Dakota 55705(US)**

(72) Inventor: **White, Thomas C., 1127 Holly Drive, Sioux Falls South Dakota 55705(US)**

(74) Representative: **Parr, Ronald Edward R.E. Parr & Co., Colman House Station Road, Knowle Solihull West Midlands B93 0HL(GB)**

ACTORUM AG

## Description

FIELD OF THE INVENTION

The invention relates to the field of ophthalmology, and particularly to devices for conducting fluids within the eye.

BACKGROUND OF THE INVENTION

In a variety of ophthalmologic procedures, treatments, and research, it is desirable to conduit fluids into, away from, or about the eye or spaces within the orbit of the eye. For example, it is frequently desirable to provide repeated and/or prolonged drug delivery into or around the eye. In the past, drug delivery techniques have involved implanting in the eye osmotic mini-pumps attached to silastic tubing. See, e.g., Miki, "A Method for Chronic Drug Infusion into the Eye", 28 Jap. J. of Ophthalmol., 140 (1984); Eliason, "An Ocular Perfusion System", 19 Invest. Ophtholmol. Vis. Sci., 102 (1980); Michelson, "Experimental Endophthalmitis Treated with an Implantable Osmotic Minipump", 97 Arch. Ophthalmol., 1345 (1979); and Miki, "Intraocular Cannula for Continuous, Chronic Drug Delivery", 103 Arch. Ophthalmol., 712 (1985).

All of these devices, however, require the implantation of a mini-pump which must be designed and prepared to deliver a specific predetermined drug desired. These devices are large and cumbersome to attach to the eye for even a few days. They give only a slow, constant infusion, being incapable of delivering a bolus, or of delivering selectively different drugs over an intermittent time period. Furthermore, such devices depend upon the presence of a pumping pressure to prevent reflux of fluid out of the cavity into which the drug is being infused. If the pump becomes detached, either purposely or accidentally, there is no mechanism to prevent extrusion of ocular fluids, potentially causing loss of pressure in and damage to the eye.

If the discharge end of a drug delivery tube is placed in a subconjunctival space but not intraocularly, healing of the tissue around the end frequently results in scarring, rendering the space non-absorbent or substantially less absorbent of fluid. This problem is also encountered with devices designed to drain excess aqueous humor from an eye having glaucoma (elevated intraocular pressure), thereby inhibiting fluid flow and causing ocular pressure to rise to potentially dangerous levels. An excellent account of the history of glaucoma surgery is found in Bick, Use of Tantalum for Ocular Drainage, Arch. Ophthalmol., 42:373-388 (1949).

In one prior art device, the exterior end of a tube extending through the wall of the eye is provided with a pressure relief valve in the form of small slits made through the wall of the tube at its end. Reference is made to Krupin, T., et al, Valve Implants in Filtering Surgery, Am. J. Ophthalmol., 81:232-235, (1976). It is reported that fairly close control over the pressure needed to open the valve may be obtained. If the exterior or distal end of the tube is inserted beneath a flap of conjunctiva or the like, of course, the valved tube is subject to the same drawbacks as the other tubes described above.

Glaucoma surgeons have discovered that when surgery fails it is usually because the "bleb", the subconjunctival drainage space created by the surgeon, has become fibrosed, causing it to shrink and become non-absorbing.

One device that has been somewhat successful in maintaining the fluid absorbency of the bleb during the healing process was described by Molteno in 1969. Molteno, "New Implant for Drainage in Glaucoma", British J. of Ophthalmol., Vol 53, p. 161 (1969). Molteno's device was made from a "stellon" brand acrylic monomer. The device consisted of two parts—a flat plate fashioned to conform to the sclera and a gutter incorporated at the point where a drainage tube met the plate to assure an even spread of drainage into the bleb. In 1979, Molteno disclosed a new device that had a biconcave base plate and a long silicone tube, which served the same function as the first device. Reference is made to Chapter 11 of Glaucoma Surgery by Luntz, M.H., Harrison, R., and Schenker, H.I. (1984), for a description of this device.

In Soviet Inventions Illustrated, Section Mechanical, Week K42, 30 November 1983, abstract No. 83-793260 P32, Derwent Publications, London, and SU-A 980 711, a device is disclosed for the introduction of medicaments into the eye by means of a capillary tube, US-A 4 554 918 discloses a pressure relieving device for removing liquid from the eye to relieve the pressure.

A need exists in the medical field for a tissue-implantable device which would operate substantially on a continuous basis to permit fluids to be infused and/or drained into and around the eye but would not be subject to the drawbacks associated with healing and scarring of tissue or the complications associated with prior implantable pumps.

SUMMARY OF THE INVENTION

The invention, in one embodiment, provides an infusion cannula for delivering fluids to various cavities of the eye. The cannula comprises a tube having first and second ends and a check valve therebetween for allowing fluid flow only toward said first end. Means is provided for securing the first end within the eye, and means is provided at the second end for attaching the cannula to a fluid source, which may be a glaucoma alleviation device, a drug infusion device, a hilt for receiving a tube from an infusion pump, or even a rubber plug for receiving a hypodermic needle or similar device. The cannula may also include a compressible reservoir and/or a plurality of one way check valves. The reservoir further may be adapted to receive a hypodermic needle directly therein.

BRIEF DESCRIPTION OF DRAWINGS

Figure 1 is a cross-section of an eye showing a partially broken away view of the device of the invention implanted in the eye;
Figure 2 is a perspective, somewhat schematic view of an eye showing an infusion device of the invention implanted therein;

Figure 3 is a broken away view of a modified embodiment of the invention;

Figure 4 is a broken away view of another modified embodiment of the invention;

Figure 5 is a broken away view of yet another modified embodiment of the invention.

Figure 6 is a broken away, schematic representation of an eye showing the positioning therein of a fluid-dissipating device;

Figure 7 is a broken away cross sectional view taken along line 7-7 of Figure 6;

Figure 8 is a broken-away cross-sectional view of another embodiment of the fluid-dissipating device;

Figure 9 is a broken away cross sectional view taken along line 9-9 of Figure 8;

Figure 10 is a plan view of another fluid-dissipating device;

Figure 11 is a broken-away cross-sectional view taken along line 11 11 of Figure 10;

Figure 12 shows another embodiment of the fluid-dissipating device;

Figure 13 shows a valve in cross-section and broken away, useful with a fluid-dissipating device.

## BEST MODE OF CARRYING OUT THE INVENTION

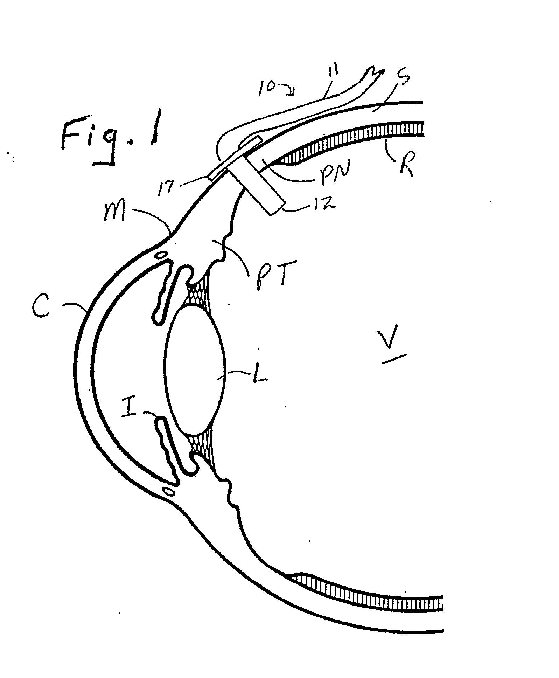

Fig. 1 shows somewhat schematically a cross-section of the human eye including a device of the invention implanted therein. In that figure the cornea is designated "C"; the iris as "I", the lens as "L", the sclera as "S", the retina as "R", the pars plicata of the ciliary body as "PT", the pars plana of the ciliary body as "PN", the vitreous cavity as "V", and the limbus as "M". An infusion cannula device (10) of the invention is shown inserted through the pars plana of the ciliary body "PN" into the vitreous cavity "V".

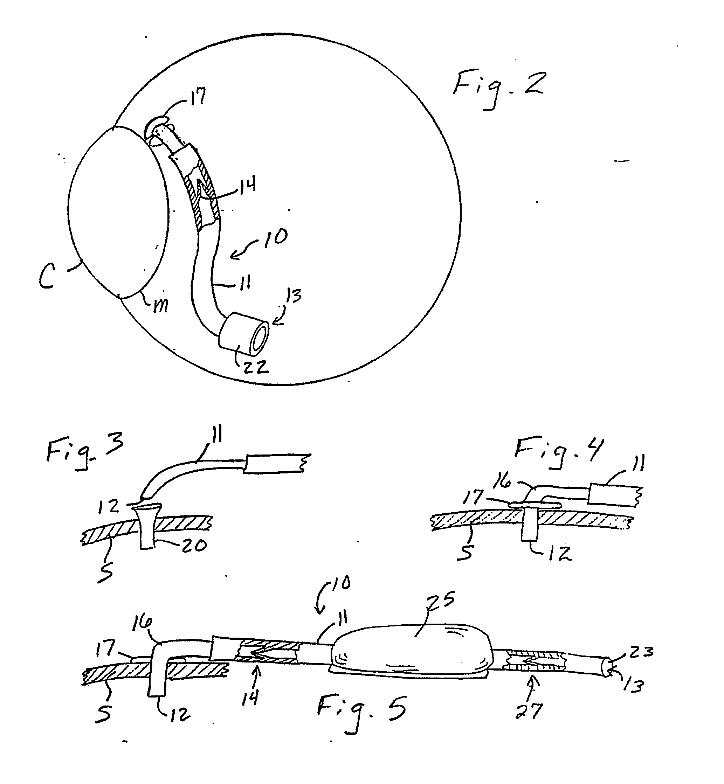

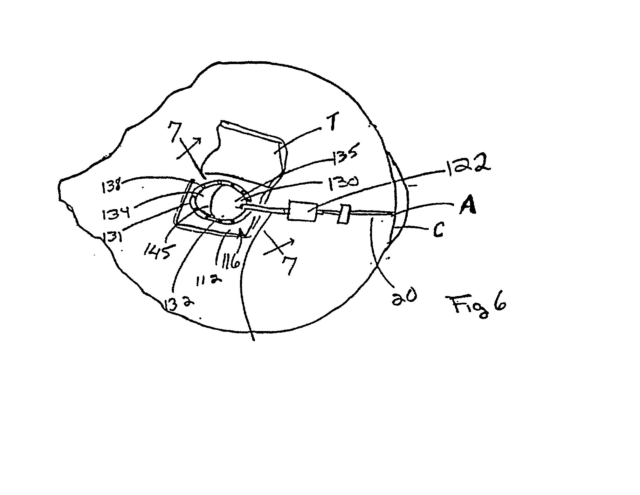

One embodiment of the device is shown more fully in Fig. 2. The cannula (10) includes a tube (11) having a first end (12) inserted through the pars plana "PN" of the ciliary body into the vitreous cavity "V". As shown in Figs. 2, 4 and 5, the first end (12) may comprise a relatively rigid tube connectable to a more flexible tube (11). The rigid portion (16) facilitates implantation and affixation of the device. The second end (13) of the tube (11) includes a hilt (22) for connection to an infusion fluid source.

A one way valve (14) is disposed within the tube between its first (12) and second (13) ends. This valve is a check valve to allow fluid flow only in a direction toward the first end, in this case being in the vitreous cavity "V" of the eye. The device may further include a flange (17) which may be secured to the sclera "S" by sutures or other conventional means.

In one surgical procedure for implanting the device (10) the conjunctiva and Tenon's capsule is opened down to bare sclera. An incision is made through the sclera into the vitreous cavity, avoiding the retina if over the pars plana or more anterior. Following this, the first end (12) is threaded through the scleral wound, and the flange (17) is secured with sutures placed in the sclera. The conjunctiva is then closed about the tube with sutures, allowing the second end (13) to come out of the conjunctiva into the cul-de-sac and exiting between the eyelids to be taped or sutured to skin of the temple.

Preferably the device is implanted through the pars plana of the ciliary body, generally about 3mm from the limbus "M". Under appropriate circumstances, however, the device could also be implanted at the limbus or cornea into the anterior chamber, or through the retina into the vitreous cavity. As previously noted, and as will be described later in greater detail, the device may also be adapted to deliver fluid into any portion of the orbital spaces, subconjunctivally, subtenonally, or retrobulbarly.

Fig. 3 shows an alternate embodiment in which a rigid trochar (20) is first implanted through the pars plana of the ciliary body, and a flexible tube is thereafter inserted through the trochar (20). The trochar (20) may then be removed, and the tube connected to the balance of the infusion cannula device (10). This technique facilitates use of entirely flexible materials, reducing the likelihood of irritation to or erosion of the conjunctiva.

For patients requiring use of the device for only a relatively short period of time, (such as several days or a few weeks) the second end (13) of the device may be position forwardly, exiting the eye between the eyelids and temporarily affixed, for example, by adhesive tape to the temple or forehead of the patient. The second end (13) may include a hilt (22) which can be selectively connected to or disconnected from complementary tubing from an infusion fluid source. Alternately, the second end (13) of the tube may comprise a solid rubber plug (23) adapted to receive fluid therethrough by injection from a hypodermic needle. In this embodiment, the tube may be small enough to position the second end (13) within the cul-de-sac of the conjunctiva. Such embodiment would be more suited to administration of infusion fluids over longer periods of time or when the patient is not hospitalized. This embodiment also has the advantage of being more aesthetically pleasing.

Fig. 5 shows another embodiment in which the device further includes a reservoir (25), and an optional second check valve (27). This embodiment is particularly suited to long term application of the device (for example, several weeks or months). When implanted, the second end (13) of the tube, which includes a rubber plug (23) for receiving a hypodermic needle, is positioned within the cul-de-sac of the conjunctiva. When administration of infusion fluid is desired, the physician inserts a hypodermic needle through the rubber plug (23) and fills the reservoir (25) with infusion fluid. The reservoir itself may be attached to the sclera "S" of the eye in a position to allow convenient digital manipulation to express the infusion fluid outwardly from the reservoir toward the first end (12) of the device (10). Alternately, the reservoir may be elastic, exerting a relatively contact compression force to slowly deliver the infusion fluid. The check valve or valves prevent reflux of the infusion fluid.

The device may be manufactured by any of a variety of well known suitable materials. Rigid portions of the device may be made from polymethylmethacrylate (PMMA) or other suitable materials such as biologically acceptable metals. The flexible portions of the tubing may be made from silicone rubber or other similar materials. The one-way check valve may be any of a variety of well known designs which need not be described in detail, but include, by way of example, well known "duck valves".

In use, the physician may surgically implant the device of the invention, as previously described, at the appropriate location in the eye. The second end (13) of the tube (11) may be draped outwardly between the eyelids and affixed for example, to the temple by adhesive tape. When infusion treatment is desired, a suitable infusion fluid source, such as an infusion pump, may be connected to the second end (13) of the tube (11), and the appropriate fluid infused. The fluid source may then be detached from the second end, the check valve preventing escape or reflux of the fluid. After an appropriate period of time, treatment may be repeated. If long term treatment is desired, the device may be provided with the appropriate structure as previously described to allow the entire device to remain within the eye and the cul-de-sac of the conjunctiva. In either case, the device of the invention provides a means for repeatedly introducing appropriate fluids into the eye without causing repeated physical invasions of the eye. The device further provides the flexibility of varying treatment from one time to the next, both as to amount and type, without replacing or disturbing the device.

Referring now to Fig. 6, an embodiment of a fluid-dissipating device used in combination with the infusion eannula of the invention is depicted. In this drawing, sclera is designated as "S"; the overlying conjunctiva and Tenon's Capsule together is designated "T"; the anterior chamber, "A"; and the cornea "C". For clarity, other structural portions of the eye have been omitted.

This embodiment of the fluid-dissipating device provides for fluid dissipation, and includes a housing (130) having walls (135) defining an interior cavity (133) positioned against or adjacent to the outer surface of the sclera "S" beneath the conjunctiva and Tenon's Capsule "T". The housing (130) is desirably made of a pliant material such as silicone rubber, a more rigid polymeric material such as polymethylmethacrylate, an inert metal such as gold, or any other convenient and biologically acceptable material. The housing (130) is typically oval or disk-shaped with a length and width typically in the range of a centimeter or smaller. The housing walls (135) defining the interior cavity (133) have sufficient rigidity to prevent substantial collapse of the cavity when the device is implanted. Located in the housing walls (135) are orifice means (145), such as small holes with diameters of at least 0.25mm that permit fluid transfer from within the interior cavity to fluid resorptive tissues (116) of the eye. The fluid resorptive tissues (116) include the sclera "S" as well as conjunctiva "T" and other adjacent tissues of the eye.

The orifice means (145) described above may include any of a variety of openings that may be located in the housing walls (135). It may be the mouth (146) of the cavity, a hole in the housing wall, (135) or multiple holes in the housing walls (135). When the device is implanted the conjunctiva and Tenon's Capsule "T" lying over the upper walls (135) of the housing do not attach to the housing (130). For this reason a space (114) exists between the tissue and the housing (130) so that, ideally, fluid flowing out of the housing (130) can envelop the housing (130), maximizing the surface area of resorptive tissue (116) available for contact with fluid. The fluid must be able to flow from the cavity to be absorbed by the tissue.

The tissue of the eye will grow around the edges of the opening. If the distance between the closest inner edges of the opening measures less than 0.25mm, the tissue growing into the opening around the edges may be able to contact other tissue and grow together, clogging the orifice means, thus effectively blocking the flow of fluid from the cavity into the surrounding tissue. In order to provide maximum drainage the orifice means should have an effective diameter of at least 0.25mm, preferably at least 0.5mm. (For purposes of this application, an orifice has an "effective diameter" of at least 0.25 mm when the orifice defines an opening extending at least 0.125mm in all directions from a point within the opening; i.e , a circle having a diameter of 0.25mm can be inserted through the orifice.)

Tube means (120) having an inner diameter substantially less than the inner diameter of the cavity (133) communicates at one end (121) with the cavity (133), and at the other end with a fluid source. The fluid source may be an artificial device attached to the eye (such as that shown as (122) in Fig. 6), the anterior chamber (15) of the eye itself, an internal or external drug infusion apparatus or other external devices. The orifice includes a rim having a surface adapted to contact fluid resorptive tissue along a locus of points defining a surface representing the closest approach of fluid resorptive tissue into the cavity. The rim should be located at least approximately 0.25mm, preferably 1.0mm, from the housing end of the tube means.

In a preferred embodiment shown in Figs. 6 and 7, the housing (130) comprises a plate member (134) and a hood member (132) peripherally connected to define an interior cavity having an inner diameter substantially greater than any inner diameter of the tube means therebetween. The plate member (134) desirably is curved to fit snugly against the eye wall. The hood member (132) extends arcuately over the plate member (134) and has a radius of curvature less than the radius of curvature of the eye wall (about 12-15mm) of a human eye, preferably less than about 8-10mm. The arcuate edge (136) of the hood member (132) typically is rounded so that the tissue of the eye that contacts the hood member, when the device is implanted in the eye, will not be injured by the edge of the hood member. In this embodiment, the orifice means is defined by the space between the plate member and the arcuate edge of the hood member and has an effective diameter of at least about 0.25mm to prevent closure by tissue. The device is implanted so that the plate member (134) contacts the sclera "S", and the exterior of the hood member (132) contacts the conjunctiva and Tenon's Capsule "T".

The plate member (134) desirably is cemented or otherwise attached to a peripheral flange (131). The flange (131) may be of silicone rubber, polymethylmethacrylate or other acceptable polymers, or other convenient biologically acceptable material, and may be fastened to the scleral wall by sutures or other means. The flange (131) may have perforations (138) to receive sutures or to permit tissue ingrowth or both.

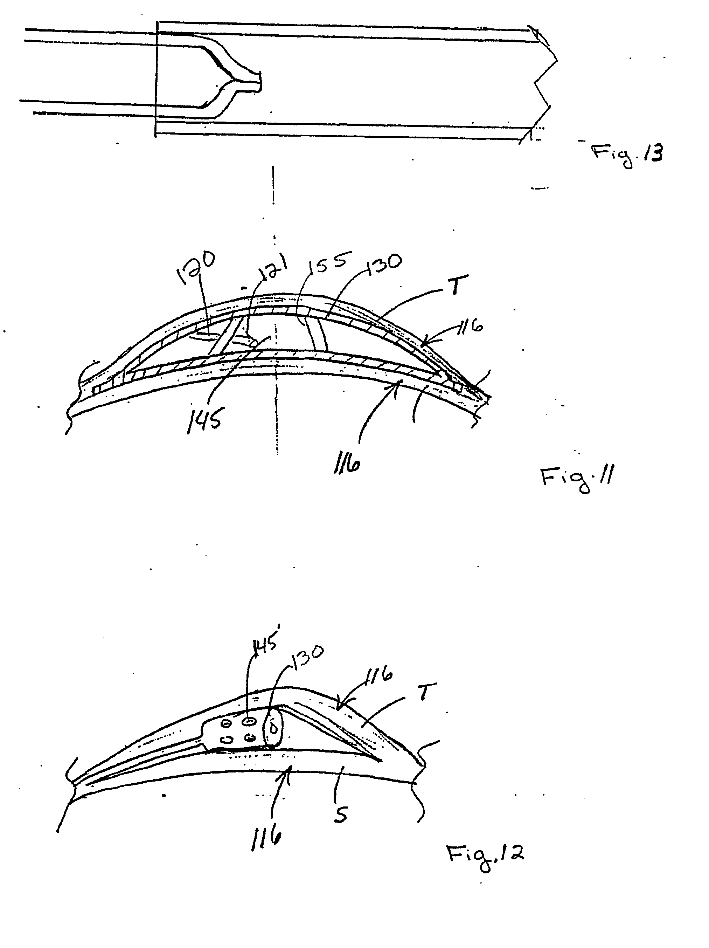

The tube means (120) preferably includes a unidirectional check valve as shown in Fig. 13 to permit fluid to flow only toward the interior cavity (133) of the housing (130) only. In the device shown in Figs. 6 and 7, the tube means (120) can establish communication with the interior cavity (133) from any position as long as the end (121) (i.e., the last portion of the tube means that has an inner cross sectional area that is substantially equal to the inner cross-sectional area of the rest of the tube means) remains at least about 0.25mm away from the orifice rim.

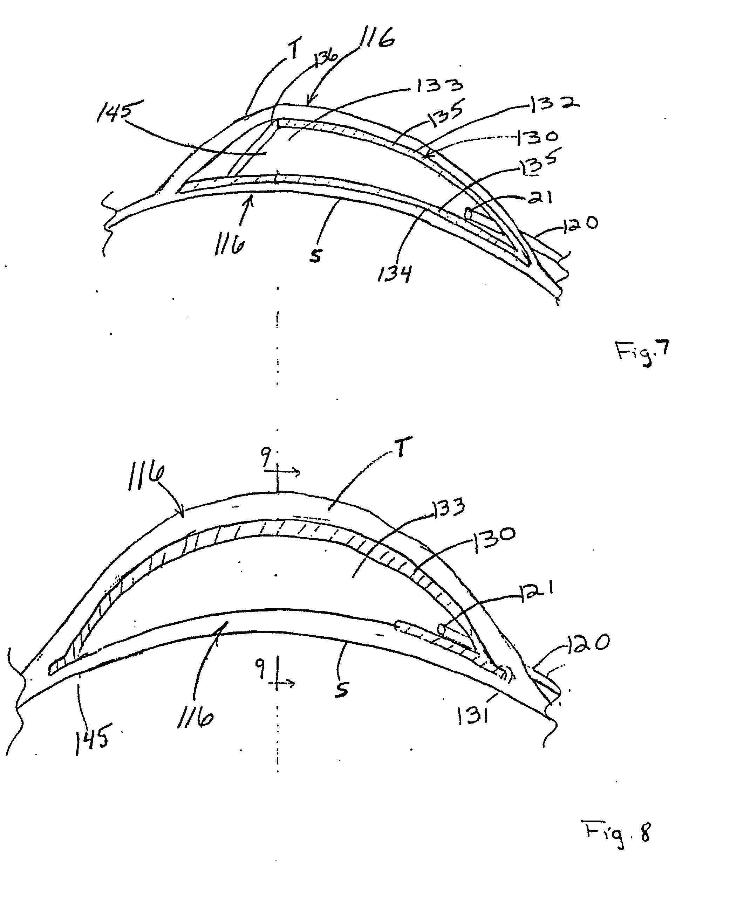

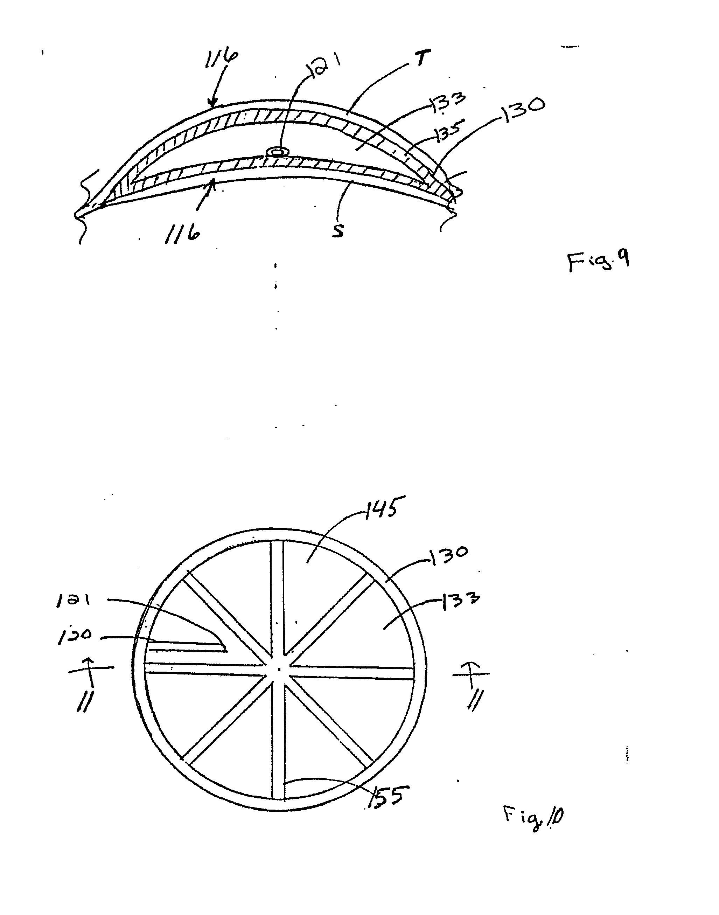

In another embodiment of the device shown in Figs. 8 and 9, the housing (130) comprises a hood member (132) having a maximum radius of curvature less than the radius of curvature of the eye wall of the human eye. The hood member (132) will thus be concave and will define an interior cavity (133) having an inner diameter substantially greater than any inner diameter of the tube means which extends between the scleral wall (112) and the interior walls of the hood member. The peripheral edge of the hood member (132) will be attached to the scleral wall. The orifice means (145) in this embodiment is defined by the inner peripheral edge (147) of the walls of the hood member, the sclera "S" of the eye providing the resorptive tissue interface to allow fluid absorption. A peripheral flange (121) may be peripherally cemented or otherwise attached to the edge of the hood member (132), or the hood member (132) may itself have a flat surface around its peripheral edge so that the hood member may be attached to the scleral wall "S" of the eye forming an interior cavity (133) within the walls (135) of the hood. Attached to an inner wall of the hood member (132) may be a plate member (134) that would permit the housing end of the tube means (120) to communicate with the cavity (133) and yet be spaced at least about 0.25mm from the sclera "S" or any other resorptive tissue in any direction. Although a plate member is shown, it would not be necessary if the tube means end communicates with the housing from the top or sides as long as the end of the tube means is kept at least about 0.25mm from the sclera.

In yet another embodiment of the fluid-dissipating device shown in Figs. 10 and 11, the housing (130) comprises a plate member (134), preferably oval or disk-shaped, having a peripheral edge to which a plurality of support structures (155) are attached. The support structures extend arcuately over the plate member and meet in a common point, defining an interior cavity (133). This device resembles a cage. The support structures (155) are of sufficient rigidity to prevent substantial collapse of the cavity when the device is implanted, i.e., although the housing may undergo some deformation, the cavity and orifice means remain open and the 0.25mm limitation with respect to the orifice means are continuously met. The open space between support structures (155) must have an effective diameter of at least about 0.25mm to prevent tissue overlying the support structures (155) from forming a bridge between the support structures (155) thereby clogging the drainage area. The tube means (120) should communicate with the housing in such a way that the support structures (155) will protect the tube means (120) from contacting resorptive tissue of the eye, thus preventing tissue from growing over the tube end and preventing fluid flow. In other words, the rim of the orifice means (the space between support structures) must be at least about 0.25mm away from the housing end of the tube means.

In still another embodiment of the fluid-dissipating device shown in Fig. 12, the housing may be an extension of the tube means. In this device the housing has walls that define a cavity having an inner diameter substantially greater than any inner diameter of the tube means and orifice means, the cavity and the orifice having a cross-sectional area available for fluid flow substantially greater than that of the interior of the tube means. The housing walls extending from the walls of the tube means must have sufficient rigidity to prevent substantial collapse of the cavity when the device is implanted. Orifice means located in the housing walls must have an effective diameter of at least about 0.25mm, and the rim must be at least about 0.25mm away from the end of the tube means.

Other types of housings may also be used so that the dissipation device serves the same purpose of protecting the end of the tube means from being clogged through contact with tissue of the eye. Such variations may include a device in which the tube means separates into two separate portions at the housing end so that there are two or more ends located within the housing. Typically the two ends will form a "T" within the housing of the device. Other variations will be apparent to the skilled artisan.

While a preferred embodiment of the present invention has been described, it should be understood that various changes, adaptations and modifications may be made therein without departing from the scope of the appended claims.

## Claims

1. An infusion cannula for delivering fluids into cavities of an eye, comprising a tube (11) having first and second ends (12, 13) and a check valve (14) therebetween for allowing fluid flow only toward said first end; means (17) for securing the first end within the eye; and means (22, 23) at said second end for attaching the cannula to a fluid source.

2. The infusion cannula of Claim 1 further comprising a compressible reservoir (25) interposed between said check valve and said second end for receiving infusion fluid.

3. The infusion cannula of Claim 1 wherein the attaching means comprises a rubber plug (23) for receiving therethrough a hypodermic needle.

4. The infusion cannula of Claim 1 wherein the securing means comprises a flange 17 extending radially outwardly from the tube near its first end, the flange being attachable to the eye.

5. The infusion cannula of Claim 1 wherein the attaching means comprises a hilt (22) for receiving infusion tubing.

6. The infusion cannula of Claim 1 further including a fluid-dissipating device at the first end of the cannula, the device comprising a housing (130) having walls (135) defining an interior cavity (133) and having sufficient rigidity to prevent substantial collapse of the cavity when the device is implanted, the walls further defining outwardly open orifice means (145) to allow fluid to flow from within the cavity directly into liquid absorbing proximity with liquid absorbent tissue, the cavity having an inner diameter substantially greater than any inner diameter of the cannula.

7. The device of Claim 6 wherein the orifice means has an effective diameter of at least about 0.25 mm.

8. The device of Claim 7 wherein the orifice means include a rim having a surface adapted to contact fluid resorptive tissue along a locus of points defining a surface representing the closest approach of fluid resorptive tissue into the cavity.

9. An infusion cannula for delivering fluids into cavities of an eye, comprising a tube (11) having first and second ends (12, 13), a check valve (14) disposed within the tube between said first and second ends for allowing fluid flow only toward said first end a comporessible reservoir (25) interposed between said check valve and the said second end for receiving infusion fluid; means (17) for securing the first end within the eye and including a flange (17) extending radially outwardly of the tube near its first end, the flange being attachable to the eye; and means (22, 23) at said second end for attaching the cannula to a fluid source.

## Patentansprüche

1. Infusionskanüle zum Einfüllen von Flüssigkeiten in Hohlräume eines Auges, mit einem Schlauch (11), der ein erstes und ein zweites Ende (12, 13) und dazwischen ein Rückschlagventil (14) aufweist, um einen Flüssigkeitsstrom nur in Richtung auf das erste Ende zuzulassen; mit Mitteln (17) zum Befestigen des ersten Endes innerhalb des Auges; und mit Mitteln (22, 23) am zweiten Ende zum Anbringen der Kanüle an einer Flüssigkeitsquelle.

2. Infusionskanüle nach Anspruch 1, gekennzeichnet durch ein kompressibles Reservoir (25) zur Aufnahme von Infusionsflüssigkeit, welches zwischen dem Rückschlagventil und dem zweiten Ende angeordnet ist.

3. Infusionskanüle nach Anspruch 1, dadurch gekennzeichnet, daß die Anbringungsmittel einen Gummistecker (23) zur Aufnahme einer Nadel für eine subkutane Spritze aufweisen.

4. Infusionskanüle nach Anspruch 1, daduch gekennzeichnet, daß die Befestigungsmittel einen Flansch (17) aufweisen, der sich von dem Schlauch in der Nähe seines ersten Endes radial nach außen erstreckt, wobei der Flansch an dem Auge anbringbar ist.

5. Infusionskanüle nach Anspruch 1, dadurch gekennzeichnet, daß die Anbringungsmittel einen Griff (22) zur Aufnahme des Infusionsschlauchs aufweisen.

6. Infusionskanüle nach Anspruch 1, gekennzeichnet durch eine flüssigkeitsaufnehmende Vorrichtung am ersten Ende der Kanüle, wobei die Einrichtung ein Gehäuse (130) mit Wänden (135) aufweist, die einen inneren Hohlraum (133) bilden und eine genügende Steifigkeit haben, um im wesentlichen ein Zusammenfallen des Hohlraums zu verhindern, wenn die Einrichtung implantiert wird, wobei die Wände außerdem nach außen geöffnete Mündungsmittel (145) bilden, um der Flüssigkeit zu ermöglichen, vom Inneren des Hohlraums unmittelbar in die Flüssigkeit absorbierende Nähe mit einem Flüssigkeit absorbierenden Gewebe zu fließen, wobei der Hohlraum einen inneren Durchmesser aufweist, der wesentlich größer als irgendein innerer Durchmesser der Kanüle ist.

7. Vorrichtung nach Anspruch 6, dadurch gekennzeichnet, daß die Mündungsmittel einen effektiven Durchmesser von mindestens ungefähr 0,25 mm aufweisen.

8. Vorrichtung nach Anspruch 7, dadurch gekennzeichnet, daß die Mündungsmittel einen Rand mit einer Oberfläche aufweisen, die so ausgebildet ist, daß sie ein Flüssigkeit resorbierendes Gewebe entlang eines geometrischen Ortes von Punkten berührt, welche eine Oberfläche bilden, die die nächste Annäherung des Flüssigkeit resorbierenden Gewebes in den Hohlraum darstellt.

9. Infusionskanüle zum Einfüllen von Flüssigkeiten in Hohlräume eines Auges, mit einem Schlauch (11), der ein erstes und ein zweites Ende (12, 13) aufweist, einem Rückschlagventil (14), welches in dem Schlauch zwischen dem ersten und dem zweiten Ende angeordnet ist, um einen Flüssigkeitsstrom nur in Richtung auf das erste Ende zuzulassen; mit einem kompressiblen Reservoir (25) zur Aufnahme von Infusionsflüssigkeit, welches zwischen dem Rückschlagventil und dem zweiten Ende angeordnet ist; mit Mitteln (17) zum Befestigen des ersten Endes innerhalb des Auges und einschließlich eines Flansches (17), der sich von dem Schlauch in der Nähe seines ersten Endes radial nach außen erstreckt, wobei der Flansch an dem Auge anbringbar ist; und mit Mitteln (22, 23) an seinem zweiten Ende zum Anbringen der Kanüle an einer Flüssigkeitsquelle.

## Revendications

1. Canule d'infusion pour distribuer des fluides dans des cavités d'un œil, comprenant:
– un tube (11) ayant des première et deuxième extrémités (12, 13) et un clapet de non-retour (14) entre les deux pour permettre un écoulement de fluide uniquement vers ladite première extrémité;
- un moyen (17) pour fixer la première extrémité à l'intérieur de l'œil; et
- un moyen (22, 23) au niveau de ladite deuxième extrémité pour connecter la canule à une source de fluide.

2. Canule d'infusion selon la revendication 1, comprenant en outre un réservoir comprimable (25) interposé entre ledit clapet de non-retour et ladite deuxième extrémité, pour recevoir du fluide d'infusion.

3. Canule d'infusion selon la revendication 1, dans laquelle le moyen de connexion comprend un bouchon en caoutchouc (23) pour recevoir à travers lui une aiguille hypodermique.

4. Canule d'infusion selon la revendication 1, dans laquelle le moyen de fixation comprend une collerette (17) s'étendant radialement vers l'extérieur à partir du tube près de sa première extrémité, la collerette pouvant être fixée sur l'œil.

5. Canule d'infusion selon la revendication 1, dans laquelle le moyen de connexion comprend un manche (22) pour recevoir le tube d'infusion.

6. Canule d'infusion selon la revendication 1 comprenant en outre un dispositif de dissipation du fluide à la première extrémité de la canule, le dispositif comprenant une enveloppe (130) ayant des parois (135) délimitant une cavité intérieure (133) et ayant une rigidité suffisante pour empêcher un affaissement substantiel de la cavité lorsque le dispositif est implanté, les parois délimitant en outre un moyen formant orifice ouvert vers l'extérieur (145) pour permettre à du fluide de s'écouler depuis l'intérieur de la cavité directement jusqu'à proximité d'absorption de liquide avec du tissu absorbant le liquide, la cavité ayant un diamètre interne substantiellement plus grand que n'importe quel diamètre interne de la canule.

7. Dispositif selon la revendication 6, dans lequel le moyen formant orifice a un diamètre utile d'au moins environ 0,25 mm.

8. Dispositif selon la revendication 7, dans lequel le moyen formant orifice comprend un rebord ayant une surface adaptée pour venir en contact avec le tissu résorbant le fluide, le long d'un lieu de points définissant une surface représentant la voie d'accès le plus proche de tissu résorbant le fluide dans la cavité.

9. Canule d'infusion pour distribuer des fluides dans des cavités d'un œil, comprenant:
- un tube (11) ayant des première et deuxième extrémités (12, 13), un clapet de non-retour (14) disposé à l'intérieur du tube entre lesdites première et deuxième extrémités pour permettre un écoulement de fluide uniquement vers ladite première extrémité;
- un réservoir comprimable (25) interposé entre ledit clapet de non-retour et ladite deuxième extrémité, pour recevoir du fluide d'infusion;
- un moyen (17) pour fixer la première extrémité à l'intérieur de l'œil et comprenant une collerette (17) s'étendant radialement vers l'extérieur du tube près de sa première extrémité, la collerette pouvant être fixée sur l'œil; et
- un moyen (22, 23) au niveau de ladite deuxième extrémité pour connecter la canule à une source de fluide.

Fig. 1

Fig. 2

Fig. 3

Fig. 4

Fig. 5

Fig 6

Fig. 7

Fig. 8

Fig. 9

Fig. 10

Fig. 13

Fig. 11

Fig. 12